# EUROPEAN PATENT APPLICATION

(11) **EP 1 647 282 A1**
(43) Date of publication of application: **19.04.2006**
(21) Application number: 05020437.9
(22) Date of filing: 20.09.2005
(51) Int. Cl.: A61K 47/10, A61K 8/00, A61K 36/00

(54) **Topical anti-inflammatory aqueous gel containing menthol, thymol and eucalyptol, process for their preparation and use of thereof**

(30) Priority: 05.10.2004 IT MI20041882
(71) Applicant: Setti, Francesco, 15053 Castelnuovo Scrivia AL (IT)
(72) Inventor: Setti, Francesco, 15053 Castelnuovo Scrivia AL (IT)
(74) Representative: Tansini, Elio Fabrizio

(57) **Abstract**

The present invention relates to antiphlogistic- and analgesic-acting acqueous pharmaceutical gel compositions.

Said compositions are utilizable for the topical treatment of inflammatory pathologies and/or painful conditions of a different origin of the human and animal body.

The compositions of the present invention include menthol, eucalyptol and thymol, in admixture or not, with other active substances of a natural origin and/or with extracts of medicinal plants, in the presence of opportune carrier agents.

## Description

The present invention relates to acqueous pharmaceutical compositions, in form of a gel, with an antiphlogistic- and analgesic action.

Said compositions are preferably utilizable for the topical treatment of inflammatory pathologies and/or painful conditions of a different origin of the human or animal body.

The compositions of the present invention include menthol, eucalyptol and thymol, in admixture or not, with other active substances of a natural origin and/or with extracts of medicinal plants, in the presence of opportune carrier agents.

The particular gel composition obtained through the preparation method of the present invention advantageously allows the synergic use of low doses of active substances.

The use of topical compositions (salves, creams, ointments, gels) containing, for example, antiinflammatory- and/or analgesic-acting active substances for treating painful traumas or pathologies of a different origin is known.

The topical compositions usually show a series of advantages with respect to the compositions for oral use (or injectable compositions).

In fact, an often prolonged oral administration of anti-inflammatory- and/or analgesic-acting substances frequently gives rise to gastrointestinal irritation and to a series of not negligible side effects (gastritis, ulcers, hepatic intoxications, sensitizations of allergic type at systemic level, sedation, alterations at cognitive level).

The topical, transcutaneous administration of active substances is able to avoid a considerable part of the risks ascribable to an oral administration.

However, it is known that the skin is a defense element of the organism. As such, it is characterized by a high impermeability/hydrophobicity and, as a consequence, it tends to avoid the trandermic passage of foreign substances from the outside towards the deepest layers of the dermis.

Furthermore, also in the case of a topical administration, it is not to be excluded, however, a possible onset of reactions of local sensitization to the drug and/or the used carriers and excipients.

So, it is not then easy to individuate and to adjust, for a determined pharmacologically active substance, a topical trandermic composition which, besides being substantially water-born (and therefore hydrophilic), ensures an effective supply of said substance to the concerned part of the human or animal body, without giving rise to undesired side effects of local sensitization.

The problem above pointed out has not yet been solved in a satisfactory way, in particular with respect to the transdermic passage of antiinflammatory- and/or analgesic-acting substances.

The technical problem faced up by the present invention is the one to ensure the organism with an adequate supply, by topical administration, of substances with an anti-inflammatory and analgesic action for the treatment of inflammatory pathologies and/or painful conditions of the human and animal body, without the occurrence of undesired local side effects.

The solution of the above technical problem has been supplied by the Applicant, which has individuated particular pharmaceutical compositions able to positively respond to the need above-described, as it will clearly result from the detailed following description.

Acqueous gel compositions (or acqueous-based gel), including a combination of menthol, eucalyptol and thymol, in the presence of proper carrier agents, then form an object of the present invention, as described and claimed in the appended claims.

Preferably, said compositions can further include one or more other active substances of a natural origin and/or extracts of medicinal plants.

In the following description, as well as in the appended claims, the quantities of the ingredients of the compositions of the invention will be expressed in weight percent based on the total weight of the composition.

The gel acqueous compositions of the present invention, including menthol, eucalyptol and thymol, are characterized in that they further include:
a quantity of a glycolic or polyhydric carrier agent,
selected from: ethylene glycol, propylene glycol or
their mixtures.

In the compositions of the present invention:
- the quantity of menthol is between 0,1% and 2%; preferably, between 0,3% and 1% by weight;
- the quantity of eucalyptol is between 0,3% and 2%; preferably, between 0,7% and 1,6% by weight;
- the quantity of thymol is between 0,1% and 2%; preferably, between 0,5% and 1,5% by weight.

The quantity of carrier agent is between 5% and 35%; preferably, between 9% and 28%; more preferably, between 12% and 24% by weight.

In the compositions of the present invention, the water is preferably demineralized water in a quantity between 45% and 80% by weight.

In a preferred embodiment, the quantity of menthol amounts to about 0,5% by weight.

In another preferred embodiment, the quantity of eucalyptol amounts to about 1% by weight.

In another preferred embodiment, the quantity of thymol amounts to about 0,8% by weight.

In a particularly preferred embodiment, the quantity of menthol amounts to 0,5%, the quantity of eucalyptol amounts to 1%, the quantity of thymol amounts to 0,8% by weight.

The compositions including, as a carrier agent, propylene glycol, are particularly preferred.

The use of the propylene glycol has allowed to limit the quantity of water employed in the preparation of the gel compositions of the present invention.

In this way, it has been possible to considerably improve the transcutaneous absorption of the active substances, without the need of adding to the composition oily or greasy ingredients, such as for example white mineral oil.

Accordingly, it has been possible to use particularly low doses of active substances, however obtaining good therapeutic results without the occurrence of undesired local sensitization reactions, even after repeated applications.

Moreover, the compositions above described have also shown an unexpected synergy of action among the used active substances.

In a particularly preferred embodiment, the quantity of propylene glycol amounts to about 15% by weight.

In another particularly preferred embodiment, the quantity of propylene glycol amounts to about 20% by weight.

Preferably, the compositions of the present invention further include:
- a quantity of at least an active substance selected from: camphor, methyl salicylate; and/or
- a quantity of at least an extract of medicinal plants selected from: arnica, *symphytum officinale*.

Preferably, the quantity of said at least an active substance is between 0,5% and 5%; more preferably, between 1% and 3% by weight.

In a preferred embodiment, the quantity of camphor amounts to about 1,5% by weight.

In another preferred embodiment, the quantity of methyl salicylate amounts to about 1,5% by weight. In a particularly preferred embodiment, the composition includes a quantity of camphor of about 1,5% by weight and a quantity of methyl salicylate of about 1,5% by weight.

Preferably, the quantity of said at least one extract of medicinal plants is between 3% and 20% by weight; more preferably, between 5% and 15% by weight.

In a preferred embodiment, the quantity of extract of arnica amounts to about 10% by weight.

In another preferred embodiment, the quantity of extract of *symphytum officinale* amounts to about 10% by weight.

In a particularly preferred embodiment, the composition includes a quantity of arnica extract of about 10% by weight and a quantity of *symphytum officinale* extract of about 10% by weight.

In another particularly preferred embodiment, the composition includes: a quantity of camphor of about 1,5% by weight, a quantity of methyl salicylate of about 1,5% by weight, a quantity of arnica extract of about 10% by weight and a quantity of *symphytum officinale* extract of about 10% by weight.

The compositions of the present invention also include other additional ingredients, such as, for example, solvents, carriers, neutralizing agents, gelling agents, stabilizers, preservatives, excipients, dyes.

Said ingredients are opportunely dosed in such a quantity to allow the preparation of stable gels, easily absorbed by the skin without leaving oily residues.

By way of absolutely not limiting example, some of the preferred ingredients, among those above-mentioned, can be selected from:
- isopropyl alcohol, preferably in a quantity between 5% and 20% by weight;
- carboxyvinyl polymers, such as carbopol (for example, Carbopol 940 and the like), preferably in a quantity of about 1% by weight;
- biocompatible amines, such as, for example, triethanolamine, in a quantity sufficient for neutralizing the acid groups of the carbopol; preferably, in a quantity between 0,8% and 1,8% by weight.

A process for the preparation of the compositions previously described forms another object of the present invention, as described and claimed in the appended claims.

The process for preparing the acqueous gel compositions of the present invention includes:
- dissolving in an alcoholic solvent a quantity of menthol, eucalyptol, thymol and a carrier agent, together, or not, with a quantity of at least an active substance selected from: camphor, methyl salicylate; and/or
   a quantity of at least an extract of medicinal plants selected from: arnica, *symphytum officinale,* to give a mixture A;
- dissolving in demineralized water a quantity of a carboxyvinyl polymer, to give a mixture B;
- dissolving in demineralized water a quantity of a biocompatible amine, in the presence, or not, of substances such as stabilizers, preservatives, excipients, dyes, to give a mixture C;
- introducing in a mixer, in the shown order, the mixtures A - B - C and keeping under stirring the resulting mixture, until the formation of the desired gel.

Preferably, the three mixtures A, B, C are separately prepared, under stirring and at a temperature close to the room temperature (preferably, not higher than 35°-40°C).

In the mixture A, the alcoholic solvent is preferably isopropyl alcohol, in the percent quantities previously described.

In the mixture B, the carboxyvinyl polymer is preferably carbopol, in the percent quantities previously described.

In the mixture C, the biocompatible amine is, preferably, triethanolamine, in the percent quantities previously described.

The ingredients of the single mixtures are added in the percent quantities previously described.

The order in which said ingredients are added in the respective mixtures is not limiting.

The mixing of A, B and C, in the shown order, is carried out under continuous stirring, at a temperature close to the room temperature (preferably, between 20°C and 30°C), for all the time neeeded for obtaining a final composition in form of a gel with the desired consistency.

The compositions of the present invention have proved to be particularly useful and effective when topically applied, both for human and veterinary use. In the veterinary field, for example, they proved to be particularly useful for alleviating the fatigue and the muscular tension which accumulate themselves in the legs of the race horses, following to a race or a training.

In the human field, they proved to be particularly useful for alleviating and/or solving a great number of phlogistic and/or painful conditions caused, for example, by arthritis, osteopathies, muscular traumas of sport and non-sport origin, peripheral neurophaties, post-operative traumas.

The compositions of the present invention have proved to be considerably effective and have generally been well tolerated by patients (they did not give rise to undesired local sensitization reactions).

Furthermore, the topical application of said compositions is resulted particularly appreciated to the user, since the same are completely absorbed from the skin without leaving any sensation of grease or residues of a different kind.

Accordingly, the use of menthol, eucalyptol and thymol forms another object of the present invention, according to what previously described and in the following sperimental section, for the preparation of acqueous pharmaceutical gel compositions for the topical treatment of inflammatory pathologies and/or painful conditions of the human or animal body, as described and claimed in the appended claims.

In a further preferred embodiment, the gel compositions of the present invention can also be dispersed/solubilized in hot water, so as to allow the preparation of a regenerating and tonic bath or foot bath.

In this way, it has been possible to effectively eliminate each fatigue and exhaustion sensation from the organism, for example after an intense sport or after a particularly heavy working day.

In a preferred embodiment, one or two coffee spoons of gel are sufficient to prepare a regenerating and tonic foot bath.

In another preferred embodiment, from two to four gel spoonfuls are preferably shown in order to prepare a regenerating and tonic complete bath.

The use of the gel compositions above-mentioned then forms a further object of the present invention, for the preparation of regenerating and tonic baths or foot baths, to effectively eliminate the fatigue and exhaustion sensation from the organism.

By way of absolutely not limiting example of the present invention, some of the particularly preferred preparations are summarized herein below.

### Example 1

In a mixer made of stainless steel, equipped with a mechanical stirring, under continuous stirring and at a varying temperature between 23°C and 27°C, a mixture A is prepared, consisting of:

| | |
|---|---|
| Isopropyl alcohol: | 10,00 kg. |
| Menthol: | 0,50 kg. |
| Eucalyptol: | 1,00 kg. |
| Thymol: | 0,80 kg. |
| Camphor: | 1,50 kg. |
| Methyl salicylate: | 1,50 kg. |
| Propylene glycol: | 20,00 kg. |

The components are added in the mixer according to the shown order.

Separately, a mixture B is prepared, under stirring and at a temperature of about 23°C, consisting of:

| | |
|---|---|
| Demineralized water: | 50,00 kg. |
| Carbopol 940: | 1,00 kg. |

In a third container, a mixture C, under stirring and at a temperature of about 23°C, is prepared, consisting of:

| | |
|---|---|
| Demineralized water: | 13,00 kg. |
| Isocide C (Methylisothiazolinone + MCIT): | 0,10 kg. |
| Bronopol (2-bromo-2-nitropropan-1,3-diol): | 0,02 kg. |
| Blue colour E 133: | 0,07 kg. |
| Yellow colour E 102: | 0,07 kg. |
| Triethanolamine: | 1,20 kg. |

The triethanolamine is added last.

The three mixtures A, B, C are maintained under stirring until complete mixing of the components, then the mixtures B and C are added, in the order, in the mixer containing the mixture A.

The resulting mixture is kept under continuous stirring, at a temperature of about 25°C, until the composition reaches the consistency of a soft gelatine, easy to spread.

### Example 2

Following the procedure of the example 1, the three following mixtures are prepared:

| Mixture A: | |
|---|---|
| Isopropyl alcohol: | 10,00 kg. |
| Menthol: | 0,50 kg. |
| Eucalyptol: | 1,00 kg. |
| Thymol: | 0,80 kg. |
| Extract of arnica 1:2: | 10,00 kg. |
| Propylene glycol: | 15,00 kg. |

| Mixture B: | |
|---|---|
| Demineralized water: | 50,00 kg. |
| Carbopol 940: | 1,00 kg. |

| Mixture C: | |
|---|---|
| Demineralized water: | 11,00 kg. |
| Isocide C: | 0,10 kg. |
| Bronopol: | 0,02 kg. |
| Red colour E 122: | 0,07 kg. |
| Triethanolamine: | 1,00 kg. |

In the mixture A, the propylen glycol is added at the beginning, together with the isopropyl alcohol.

The three mixtures A, B and C are mixed as described in the example 1, to give the final gel compound.

### Example 3

Following the procedure of the example 1, the three following mixtures are prepared:

| Mixture A: | |
|---|---|
| Isopropyl alcohol: | 10,00 kg. |
| Menthol: | 0,50 kg. |
| Eucalyptol: | 1,00 kg. |
| Thymol: | 0,80 kg. |
| Extract of *symphytum officinale:* | 10,00 kg. |
| Propylene glycol: | 15,00 kg. |

| Mixture B: | |
|---|---|
| Demineralized water: | 50,00 kg. |
| Carbopol 940: | 1,00 kg. |

| Mixture C: | |
|---|---|
| Demineralized water: | 11,00 kg. |
| Isocide C: | 0,10 kg. |
| Bronopol: | 0,02 kg. |
| Red colour E 122: | 0,03 kg. |
| Triethanolamine: | 1,00 kg. |

In the mixture A, the propylene glycol is added at the beginning, together with the isopropyl alcohol.

The three mixtures A, B and C are mixed as described in the example 1, to give the final gel compound.

## Claims

1. Acqueous pharmaceutical gel compositions, including: menthol, eucalyptol and thymol, **characterized in that** they further include:
a quantity of a glycolic or polyhydric carrier agent, selected from: ethylene glycol, propylene glycol, glycerol or their mixtures.

2. Compositions according to claim 1, wherein:
the quantity of menthol is between 0,1% and 2% by weight;
the quantity of eucalyptol is between 0,3% and 2% by weight;
the quantity of thymol is between 0,1% and 2% by weight.

3. Compositions according to claim 1, wherein the quantity of carrier agent is between 5% and 35% by weight, preferably between 9% and 28%.

4. Compositions according to claim 3, wherein said carrier agent is propylene glycol.

5. Compositions according to claim 1, including demineralized water in a quantity between 45% and 80% by weight.

6. Compositions according to any one of the claims 1-5, further including:
a quantity of at least an active substance selected from: camphor, methyl salicylate; and/or
a quantity of at least an extract of medicinal plants selected from: arnica, *symphytum officinale*.

7. Compositions according to claim 6, wherein:
the quantity of said at least one active substance is between 0,5% and 5% by weight; preferably, between 1% and 3%;
the quantity of said at least one extract of medicinal plants is between 3% and 20% by weight; preferably, between 5% and 15%.

8. Compositions according to claims 6-7, wherein:
the quantity of camphor is about 1,5% by weight;
the quantity of methyl salicylate is about 1,5% by weight;
the quantity of extract of arnica is about 10% by weight;
the quantity of extract of *symphytum officinale* is about 10% by weight.

9. Compositions according to any one of the claims 1-8, further including ingredients, such as solvents, carriers, gelling agents, neutralizing agents, stabilizers, preservatives, excipients, dyes.

10. Compositions according to claim 9, wherein:
the solvent is isopropyl alcohol in a quantity between 5% and 20% by weight;
the gelling agent is a carboxyvinyl polymer, such as carbopol, in a quantity of about 1% by weight;
the neutralizing agent is a biocompatible amine, such as triethanolamine, in a quantity between 0,8% and 1,8% by weight.

11. Process for the preparation of a composition according to any one of the claims 1-10, including:
dissolving in an alcoholic solvent a quantity of menthol, eucalyptol, thymol and a carrier agent, together, or not, with:
a quantity of at least an active substance selected from: camphor, methyl salicylate; and/or
a quantity of at least an extract of medicinal plants selected from: arnica, *symphytum officinale,* to give a mixture A;
dissolving in demineralized water a quantity of a carboxyvinyl polymer, to give a mixture B;
dissolving in demineralized water a quantity of a biocompatible amine, in the presence, or not, of substances such as stabilizers, preservatives, excipients, dyes, to give a mixture C;
introducing in a mixer, in the shown order, the mixtures A - B - C and keeping under stirring the resulting mixture, until formation of the gel.

12. Process according to claim 11, wherein:
in the mixture A, the carrier agent is propylene glycol and the alcoholic solvent is isopropyl alcohol;
in the mixture B, the carboxyvinyl polymer is carbopol;
in the mixture C, the biocompatible amine is triethanolamine.

13. Process according to claims 11-12, wherein the ingredients of the three mixtures A, B, C are dosed according to the percent quantities shown in the preceding claims.

14. Process according to any one of the claims 11-13, wherein the three mixtures A, B, C are separately prepared under stirring and at a temperature close to the room temperature.

15. Process according to claim 11, wherein the mixing A - B - C is carried out under continuous stirring at a temperature close to the room temperature.

16. Use of menthol, eucalyptol, thymol and a carrier agent according to claim 1, for the preparation of an acqueous pharmaceutical gel composition for the topical treatment of inflammatory pathologies and/or painful conditions of the human or animal body.

17. Use according to claim 16, wherein said composition is according to the claims 2-10.

18. Use of a composition according to any one of the claims 1-10 for the preparation of a regenerating and tonic bath or a foot bath.
